# FASCICULE DE BREVET EUROPEEN

(11) **EP 2 416 768 B1**
(45) Date de publication et mention de la délivrance du brevet: **07.10.2015**
(21) Numéro de dépôt: 10723231.6
(22) Date de dépôt: 09.04.2010
(51) Int. Cl.: A61K 31/192, A61K 9/00, A61K 47/48

(54) **COMPOSITIONS COMPRENANT AU MOINS UN COMPLEXE COMPOSE D'UN DERIVE D'ACIDE NAPHTOÏQUE ET D'AU MOINS UNE CYCLODEXTRINE ET LEURS UTILISATIONS**
ZUSAMMENSETZUNGEN MIT MINDESTENS EINEM KOMPLEX AUS EINEM NAPHTHOESÄUREDERIVAT UND MINDESTENS EINEM CYCLODEXTRIN SOWIE IHRE VERWENDUNG
COMPOSITIONS COMPRISING AT LEAST ONE COMPLEX COMPOSED OF A DERIVATIVE OF NAPHTHOIC ACID AND OF AT LEAST ONE CYCLODEXTRIN AND USES THEREOF

(30) Priorité: 09.04.2009 FR 0952336
(43) Date de publication de la demande: 15.02.2012
(73) Titulaire: Galderma Research & Development, 06410 Biot (FR)
(72) Inventeur: AT, Emmanuelle, F-06600 Antibes (FR); MALLARD, Claire, F-06250 Mougins (FR)
(74) Mandataire: Casalonga
(86) Numéro de dépôt international: PCT/FR2010/050681
(87) Numéro de publication internationale: WO 2010/116098

(56) Documents cités:
- WO-A-2006/070093

## Description

La présente invention concerne des compositions pour application topique, et leurs utilisations en tant que produits cosmétiques ou pharmaceutiques, lesdites compositions étant destinées, en particulier, au traitement de l'acné.

L'acné est une pathologie multifactorielle fréquente qui atteint la peau riche en glandes sébacées (visage, région scapulaire, bras et régions intertrigineuses). Elle est la plus fréquente des dermatoses. Les cinq facteurs pathogéniques suivants jouent un rôle déterminant dans la constitution de l'acné
1. la prédisposition génétique;
2. la surproduction de sébum (séborrhée);
3. les androgènes;
4. les troubles de la kératinisation folliculaire (comédogénèse); et
5. la colonisation bactérienne et les facteurs inflammatoires.

Il existe plusieurs formes d'acnés, ayant toutes en commun l'atteinte des follicules pilosébacés. On peut citer notamment, l'acné conglobata, l'acné chéloïde de la nuque, l'acné médicamenteuse, l'acné miliaire récidivante, l'acné nécrotique, l'acné neonatorum, l'acné prémenstruelle, l'acné professionnelle, l'acné rosacée, l'acné sénile, l'acné solaire, et l'acné vulgaire.

L'acné vulgaire, appelée également acné juvénile polymorphe, est la plus courante. Elle comprend quatre stades :
- Le stade 1 correspond à l'acné comédonienne caractérisée par un grand nombre de comédons ouverts et/ou fermés, et de microkystes.
- Le stade 2, ou acné papulopustuleuse, est de gravité légère à modérée. Elle est caractérisée par la présence de comédons ouverts et/ou fermés, de microkystes, mais également de papules rouges et de pustules. Elle touche principalement le visage et laisse peu de cicatrices.
- Le stade 3, ou acné papulocomédonienne, est plus grave et s'étend au dos, au thorax et aux épaules. Elle est accompagnée d'un plus grand nombre de cicatrices.
- Le stade 4, ou acné nodulokystique, s'accompagne de nombreuses cicatrices. Elle présente des nodules ainsi que des pustules volumineuses violacées et douloureuses.

Les différentes formes d'acné décrites précédemment peuvent être traitées par des actifs tels que les anti-séborrhéiques et les anti-infectieux, par exemple le peroxyde de benzoyle (notamment le produit Eclaran® commercialisé par la société Pierre Fabre), par des rétinoïdes tels que la trétinoïne (notamment le produit Retacnyl® commercialisé par la société Galderma) ou l'isotrétinoïne (produit Roaccutane® commercialisé par les Laboratoires Roche), ou encore par des dérivés d'acide naphtoïque. Les dérivés d'acide naphtoïque, tels que notamment l'acide 6-[3-(1-adamantyl)-4-méthoxyphényl]-2-naphtoïque), communément appelé adapalène (produit Differine® commercialisé par la société Galderma), sont largement décrits et reconnus comme des principes actifs aussi efficaces que la trétinoïne pour le traitement de l'acné.

L'adapalène en particulier présente une efficacité unanimement avérée ; cependant, il serait avantageux et utile que sa tolérance par voie topique, bien que supérieure à celle de ses concurrents appartenant à la même classe chimique (trétinoïne, tazarotène), soit améliorée.

L'Adapalène contenu actuellement dans les formulations de type gel ou crème est sous forme dispersé. En effet, les différents composants présents dans la composition du gel ou de la crème ne permettent pas de solubiliser l'Adapalène à une teneur de 0,1% et 0,3% (m/m). Il est donc nécessaire d'introduire dans la composition des composés permettant de solubiliser l'Adapalène.

Or, la Demanderesse a réussi à formuler l'Adapalène sous forme de complexes à base de cyclodextrines permettant de solubiliser ce principe actif dans une formulation. Le complexe Adapalène/cyclodextrine est obtenu préalablement par la technique de fluide dense sous pression et en particulier du CO₂ supercritique. Cette technique est basée sur le pouvoir solvant du CO₂ qui est modulable en fonction des conditions de pression et de température et dont le principe technique est décrit dans la demande WO2004/096284.

Une fois le complexe Adapalène/cyclodextrine obtenu sous forme de poudre, il faut réussir à stabiliser ce complexe en solution. En effet, une fois dans l'eau le complexe Adapalène/cyclodextrine se décomplexe rapidement et on observe une précipitation de l'Adapalène.

Le problème majeur à résoudre est donc de trouver une formulation où le complexe solubilisé sera stabilisé. En effet, le critère majeur qui conduit à la sélection de formulations est la décomplexation de l'Adapalène qui doit avoir lieu sur la peau et non dans la formulation.

Pour résoudre le problème technique, la demanderesse a mis au point des solutions nécessaires à la stabilisation du complexe une fois mis en solution, mais aussi pour formuler des formes galéniques, comme des gels afin d'obtenir une teneur en Adapalène de 0,1% ou de 0,3% (m/m) dans lesquels le complexe Adapalène/cyclodextrine reste stable physiquement et chimiquement.

Une fois le complexe Adaplène/cyclodextrine mis en oeuvre sous forme solubilisée dans une formulation, les exemples montrent un effet quant à la cinétique et donc une amélioration de pénétration d'un tel complexe, ainsi qu'un effet bénéfique sur l'activité comédolytique.

En voie topique, les applications pharmaceutiques des cyclodextrines sont nettement plus rares (Glymesason®), voire inexistantes dans le cadre du traitement de l'acné.

L'utilisation des cyclodextrines avec les rétinoïdes est déjà décrite avec pour objectifs principaux l'augmentation de la solubilité et de la photostabilité notamment du rétinol. Peu d'articles ou de brevets mentionnent l'usage des cyclodextrines avec pour objectif d'améliorer la pénétration cutanée ainsi que l'activité comédolytique liée à l'acné.

La demande WO2006/070093 décrit une composition comprenant de l'adapalene solubilisé en milieux aqueux avec des cyclodextrines et comprend donc le mélange physique d'adapalene et de cyclodextrine dans un milieu aqueux sans le pouvoir solvant d'un fluide dense sous pression.

Ainsi la présente invention se distingue de l'art antérieur dans le sens où elle consiste à formuler l'Adapalène sous forme de complexes à base de cyclodextrines permettant de solubiliser ce principe actif dans une formulation contenant 0.1 et 0.3% (m/m) d'Adapalène. L'Adapalène est complexé par la cyclodextrine sans étape de salification, la fonction acide de l'Adapalène est ici conservée.

La présente invention a donc pour objet une composition topique , notamment pharmaceutique, et de préférence dermatologique, comprenant, dans un milieu physiologiquement acceptable, au moins un complexe formé avec au moins un composé dérivé de l'acide naphtoïque de formule (I) ci-après, leurs sels et esters, en une concentration comprise entre 0,001% et 10% en poids, et une méthyl-β-cyclodextrine.

De préférence, ladite composition ne comprend aucun agent dépigmentant.

En particulier, ladite composition ne comprend aucun agent dépigmentant distinct du composé dérivé de l'acide naphtoïque, notamment adapalène.

Par milieu physiologiquement acceptable, on entend un milieu compatible avec la peau, les muqueuses et/ou les phanères.

Le composé dérivé de l'acide naphtoïque selon l'invention correspond au composé de formule (I), leurs sels et esters: 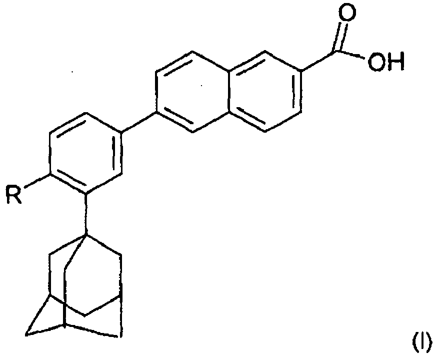 où R représente un atome d'hydrogène, un radical hydroxyle, un radical alkyle, ramifié ou non, ayant de 1 à 4 atomes de carbone, un radical alkoxy ayant de 1 à 10 atomes de carbone ou un radical cycloaliphatique substitué ou non.

Par radical alkyle linéaire ou ramifié ayant de 1 à 4 atomes de carbone, on entend de $référence les radicaux méthyle, éthyle, propyle et butyle.

Par radical alkoxy ayant de 1 à 10 atomes de carbone, on entend de préférence les radicaux méthoxy, éthoxy, propoxy, butoxy, hexyloxy et décyloxy.

Par radical cycloaliphatique, on entend de préférence les radicaux mono ou polycyclique tel que le radical methyl-1 cyclohexyle ou le radical 1-adamantyle.

Par sels des dérivés d'acide naphtoïque, on entend des sels formés avec une base pharmaceutiquement acceptable, notamment une base minérale telle que la soude, la potasse et l'ammoniaque ou une base organique telle que la lysine, l'arginine, la N-méthyl-glucamine, mais également les sels formés avec des amines grasses telles que la dioctylamine, l'aminométhyl propanol et la stéarylamine.

Par esters des dérivés d'acide naphtoïque, on entend des esters formés avec des alcools pharmaceutiquement acceptables.

De préférence, parmi les dérivés de l'acide naphtoïque susceptibles d'entrer dans les compositions selon l'invention, on choisira l'acide 6-[3-(1-adamantyl)-4-methoxyphenyl]-2-naphtoïque (adapalène), l'acide 6-[3-(1-adamantyl)-4-hydroxyphenyl]-2-naphtoïque, l'acide 6-[3-(1-adamantyl)-4-decyloxyphenyl]-2-naphtoïque ou l'acide 6-[3-(1-adamantyl)-4-hexyloxyphenyl]-2-naphtoïque,

Encore plus préférentiellement, le composé dérivé de l'acide naphtoïque utilisable selon l'invention est choisi parmi l'adapalène (l'acide 6-[3-(1-adamantyl)-4-méthoxyphényl]-2-naphtoique), ses sels et ses esters.
Par sels d'adapalène, on entend notamment les sels formés avec une base pharmaceutiquement acceptable, notamment des bases minérales telles que la soude, la potasse et l'ammoniaque ou des bases organiques telles que la lysine, l'arginine, la N-méthyl-glucamine.
On entend également par sels de l'adapalène les sels formés avec des amines grasses telles que la dioctylamine, l'aminométhyl propanol et la stéarylamine.

De préférence, le composé dérivé de l'acide naphtoïque est l'adapalène.

Dans les compositions selon l'invention, la concentration en composé dérivé de l'acide naphtoique selon la structure générale (I) mentionnée ci-dessus est comprise entre 0,001% et 10%, préférentiellement entre 0,01% et 5% et, plus préférentiellement, entre 0,05% et 2% en poids du poids total de la composition. Dans l'ensemble du présent texte, à moins qu'il ne soit spécifié autrement, il est entendu que lorsque des intervalles de concentrations sont donnés, ils incluent les bornes supérieure et inférieure dudit intervalle.

De préférence, la concentration en composé dérivé de l'acide naphtoïque est égale à 0,1%. De façon alternative, la concentration en composé rétinoïde est de préférence égale à 0,3%.

Les cyclodextrines (CD) sont des oligosaccharides cycliques constitués d'unités (α-1,4) α-D-glucopyranose avec une cavité centrale lipophile et une surface externe hydrophile (Frömming KH, Szejtli J : « Cyclodextrins in pharmacy », Kluwer Academic Publishers, Dortrecht, 1994). Les cyclodextrines sont connues pour augmenter la solubilité de molécules par la formation d'une structure en forme de « cage » possédant une partie hydrophile externe et une partie hydrophobe interne. Les cyclodextrines peuvent ainsi former des complexes d'inclusion avec beaucoup de médicaments en acceptant à l'interieur de la cavité la molécule entière, ou plus communément la partie lipophile de la molécule.

Les cyclodextrines naturelles les plus abondantes sont les α-cyclodextrines, les β-cyclodextrines et les γ-cyclodextrines.
Les α-cyclodextrines (connues également sous le nom de Schardinger's α-dextrin, cyclomaltohexaose, cyclohexaglucan, cyclohexaamylose, α-CD, ACD, C6A) comprennent 6 unités de glucopyranose. Les β-cyclodextrines (connues également sous le nom Schardinger's β-dextrin, cyclomaltoheptaose, cycloheptaglucan, cycloheptaamylose, β-CD, BCD, C7A) comprennent 7 unités de glucopyranose, et les γ-cyclodextrines (connues également sous le nom Schardinger's γ-dextrin, cyclomaltooctaose, cyclooctaglucan, cyclooctaamylose, γ-CD, GCD, C8A) comprennent 8 unités de glucopyranose.
Parmi ces trois types de CDs, les méthyl-β-cyclodextrines apparaissent comme les agents pharmaceutiques complexants utiles pour la mise en oeuvre de la présente invention, en raison de la taille de leur cavité, de leur disponibilité, de leurs propriétés et de leur faible coût.

Selon Dr J. Szejtli (« Cyclodextrins », in Encyclopedia of Supramolecular Chemistry, eds. Marcel Dekker, 2004) les cyclodextrines sont avantageuses mais présentent également des facteurs limitants qui restreignent l'application des cyclodextrines à certains types de produits pharmaceutiques. Par ailleurs, tous les produits ne sont pas appropriés pour une complexation avec des cyclodextrines. Beaucoup de produits ne peuvent pas être complexés ou bien la complexation ne procure aucun avantage essentiel. Les composés inorganiques sont en général inappropriés à la complexation avec des cyclodextrines.

Les cyclodextrines sont des composés largement utilisés pour répondre à des problématiques de solubilisation et elles sont particulièrement adaptées aux composés très peu solubles dans l'eau, comme Adapalène (Didja et al. ; Int J Pharm 54 (1989) 175-179 ; Didja et al. ; Int J Pharm 111 (1994) 111-116 ; Loftsson et al. ; Int J Pharm 115 (1995) 255-258 ; Liu et al. ; J Pharm Sci vol92 (12) 2003 2449-2457).
Les cyclodextrines sont largement utilisées pour solubiliser les principes actifs très peu solubles. Les complexes les plus fréquemment rencontrés sont à base de stéroïdes ou d'anti-inflammatoires non stéroïdiens avec des applications principalement en voie orale, parentérale ou ophtalmique.

De préférence, la méthyl-β-cyclodextrine employée dans l'invention est choisie parmi les TRIMEB (heptakis (2,3,6-trimethyl)-β-CD), les DIMEB (heptakis(2,6-dimethyl)-β-CD)ou encore les RAMEB (Randomly Methylated β-Cyclodextrine).
Plus préférentiellement, les cyclodextrines utilisées sont celles de type RAMEB (Randomly Methylated β-Cyclodextrine).

Selon un mode particulièrement préféré de l'invention, la technique utilisée pour complexer l'Adapalène est celle du CO₂ supercritique. Une technique basée sur le pouvoir solvant du CO₂ qui est modulable en fonction des conditions de pression et de température. La cyclodextrine utilisée est une méthyl-β-cyclodextrine. Le complexe, une fois préparé, est incorporé dans un gel à la teneur de 0.1% m/m et 0.3% m/m de titre d'Adapalène.

Les compositions selon la présente invention peuvent se présenter sous toutes les formes galéniques normalement utilisées pour une application topique, notamment sous forme de dispersions aqueuses, hydroalcooliques ou huileuses, de dispersions du type lotion, de gels aqueux, anhydres ou lipophiles, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-liquide ou solide du type crème, gel-crème, mousse ou pommade ou de micro émulsions, de micro capsules, de micro particules ou de dispersions vésiculaires de type ionique et/ou non ionique, ou encore sous forme de sprays.

De préférence, les compositions se présentent sous la forme d'un gel.

L'homme du métier veillera à choisir les excipients constituant les compositions selon l'invention en fonction de la forme galénique souhaitée et de manière à ce que les propriétés avantageuses de la composition selon l'invention soient respectées.

La composition selon l'invention peut en outre comprendre un ou plusieurs des ingrédients suivants :
a) un ou plusieurs agents gélifiants,
b) un ou plusieurs agents chélatants,
c) un ou plusieurs agents émollients,
d) un ou plusieurs agents conservateurs.

A titre d'exemple non limitatif de gélifiants et/ou gélifiants pH-indépendants pouvant entrer dans les compositions selon l'invention, on peut citer les polymères d'acide poly(acrylique) tels que le Carbopol 980NF, l'Acrylates/C10-30 Alkyl Acrylate Crosspolymer vendu sous le nom de Pemulen TR-1 ou Pemulen TR-2 par la société Noveon, les carbomers dits non sensibles aux électrolytes, vendus sous le nom d'Ultrez 20®, d'Ultrez 10®, de Carbopol 1382® ou de Carbopol ETD2020NF® par la Société Noveon, les polysaccharides avec à titre d'exemples non limitatifs les chitosans, la gomme de xanthane telle que le Xantural1980® vendu par la société Kelco, la gomme guar, la cellulose et ses dérivés tel que la microcrystalline cellulose et carboxymethyl cellulose de sodium vendue sous le nom d'Avicel CL-611 par la société FMC Biopolymer, l'hydroxypropylmethylcellulose en particulier le produit vendu sous le nom de Methocel E4M premium par la société Dow Chemical ou l'hydroxyéthylcellulose, en particulier, le produit vendu sous le nom de Natrosol HHX 250® par la société Aqualon, la famille des aluminium magnésium silicates tel que le Veegum K vendu par la société Vanderbilt ,la famille des polymères acryliques couplés à des chaînes hydrophobes tel que le PEG-150/decyl/SMDI copolymer vendu sous le nom de Aculyn 44 (polycondensat comprenant au moins comme éléments, un polyéthylèneglycol à 150 ou 180 moles d'oxyde d'éthylène, de l'alcool décylique et du méthylène bis(4-cyclohexylisocyanate) (SMDI), à 35% en poids dans un mélange de propylèneglycol (39%) et d'eau (26%)), la famille des amidons modifiés tels que l'amidon de pomme de terre modifié vendu sous le nom de Structure Solanace ou bien leurs mélanges et les gélifiants de la famille des polyacrylamides tels que le mélange Sodium acryloyldimethyltaurate copolymer / isohexadecane / polysorbate 80 vendu sous le nom Sepineo P 600 ® (ou de Simulgel 600 PHA®) par la société Seppic, le mélange polyacrylamide / isoparaffine C13-14 / laureth-7 comme, par exemple, celui vendu sous le nom de Sepigel 305 par la société Seppic, la famille des carraghénanes en particulier réparties sous quatre grandes familles : κ, λ, β, ω tel que les Viscarin® et les Gelcarin® commercialisés par la société IMCD

A titre d'agent gélifiant préféré, on peut citer le polymère d'acide (poly)acrylique vendu notamment sous le nom de Carbopol 980NF® par Noevon.

Parmi les agents chélatants, on peut citer à titre d'exemples non limitatifs l'acide éthylène diamine tétra-acétique (EDTA), l'acide diéthylène triamine penta-acétique (DTPA), l'acide éthylène diamine-di (O-hydroxyphényl acétique) (EDDHA), l'acide hydroxy-2-éthylène diamine triacétique (HEDTA), l'acide éthyldiamine-di (O-hydroxy-p-méthyl phényl) acétique (EDDHMA) et l'acide éthylène diamine-di (5-carboxy-2-hydroxyphényl) acétique (EDDCHA).

A titre d'agent chélatant préféré, on peut citer l'acide éthylène diamine tétra-acétique (EDTA) vendu notamment sous le nom Titriplex III®,

Parmi les agents humectants et/ou émollients qui ont pour rôle d'hydrater la peau et de faciliter l'application de la formulation, on utilise préférentiellement, sans que cette liste soit limitative, des composés tels que la glycérine, le propylène glycol ou propane-diol 1,2, le dipropylène glycol, le propylène glycol dipélargonate, le lauroglycol, seuls ou en mélange, le docusate de sodium, le sorbitol, les sucres (à titre d'exemple le glucose, le lactose), les PEG (à titre d'exemple le Lutrol E400), l'urée, les acides aminés (à titre d'exemple la sérine, la citrulline, l'alanine).

A titre d'agent humectant et/ou émollient préféré, on peut citer la glycérine, le propylène glycol et le docusate de sodium.

Parmi les agents conservateurs, on peut citer à titre d'exemples non limitatifs l'acide benzoïque et ses dérivés avec l'alcool benzylique, le chlorure de benzalkonium, le benzoate de sodium, le bronopol, la chlorhexidine, le chlorocrésol et ses dérivés, l'alcool éthylique, l'alcool phénéthylique, le phénoxyéthanol, le sorbate de potassium, la diazolidinylurée, les parabènes tels que le propyl parabène ou le méthyl parabène, pris seuls ou en mélanges.

A titre d'agent conservateur préféré, on peut citer les parabènes et le phénoxyéthanol ou le chlorure de benzalkonium seuls ou en mélange.

La composition selon l'invention peut comprendre également un ou plusieurs émulsionnants.

Les émulsionnants sont des composés amphiphiles qui possèdent une partie hydrophobe ayant une affinité pour l'huile et une partie hydrophile ayant une affinité pour l'eau créant ainsi un lien entre les deux phases. Les émulsionnants ioniques ou non ioniques stabilisent donc les émulsions huile/eau en s'adsorbant à l'interface et en formant des couches lamellaires de cristaux liquides.

Le pouvoir émulsfiant des émulsionnants non-ioniques est étroitement lié à la polarité de la molécule. Cette polarité est définie par la HLB (Balance Hydrophile/Lipophile).

Une HLB élevée indique que la fraction hydrophile est prédominante, et, à l'inverse, une faible HLB indique que la partie lipophile est prédominante. Par exemple, des valeurs de HLB supérieures à environ 10 correspondent à des tensioactifs hydrophiles.

Les émulsionnants peuvent être classés, selon leur structure, sous les termes génériques "ioniques" (anioniques, cationiques, amphotères) ou "non ioniques". Les émulsionnants non ioniques sont des émulsionnants qui ne se dissocient pas en ions dans l'eau et sont donc insensibles aux variations de pH.

On peut citer, à titre d'exemple non limitatif des émulsionnants non ioniques présentant une HLB élevée, les esters de sorbitan tels que le POE(20) sorbitan monooléate, vendu sous le nom de Tween 80 ® (HLB=15) ; le POE(20) sorbitan monostéarate vendu sous le nom de Tween 60 ® (HLB=14.9); les éthers d'alcools gras tels que le POE (21) stéaryl ether (HLB= 15.5) vendu sous le nom de Brij 721® par la société Uniqema, ou le ceteareth 20 vendu sous le nom de Eumulgin B2® (HLB de 15,5) par la société Cognis, les esters de polyoxyethylene glycol tel que le glyceryl stearate and PEG 100 stéarate vendu sous le nom de Arlacel 165 FL ® (HLB=11) par la société Uniqema , le PEG 6 Stéarate et PEG 32 stéarate vendu sous le nom de TEFOSE 1500 ® (HLB= 10) par la société Gateffossé, les sucroesters de haut HLB tel que le PEG 20 methyl glucose sesquistéarate vendu sous les noms de glucamate SSE20 (HLB=15) par la société Amerchol et le sucrose laurate vendu sous le nom de Surfhope C-1216® (HLB=16) et le sucrose stéarate vendu sous le nom de Surfhope C-1811® (HLB=11) par la société Gattefossé De préférence, lesdits émulsionnants non ioniques de haute HLB, présentent une HLB comprise entre 10 et 18.

On citera, comme exemples non limitatifs les émulsionnants non ioniques de basse HLB (lipophiles), les esters de sorbitan, tels que le monostéarate de sorbitan (HLB = 4.7) vendu sous le nom de Span 60 par la société Uniqema, les esters de glycérol tel que le monostéarate de glycerol vendu sous le nom de Cutina GMSVPH (HLB=3.8) par la sociéta Cognis, les esters de polyethylène glycol tel que le PEG-6 isostéarate vendu sous le nom de Olepal isostéarique® (HLB=8) par la société Gattefossé, les sucroesters de bas HLB tel que le methyl glucose sesquistéarate vendu sous le nom de Glucate SS® (HLB= 6) par la société Amerchol et le sucrose dilaurate vendu sous le nom de Surfhope C-1205 (HLB=5) et le sucrose tristéarate vendu sous le nom de Surfhope C-1803® (HLB=3) par la société Gattefossé.

On peut aussi citer comme autres agents émulsionnants non-ioniques, des cires auto-émulsionnantes qui permettent d'obtenir des émulsions stables facilement par simple dispersion à chaud. A titre d'exemple, le cetearyl alcohol (and) polysorbate 60 vendu sous le nom de Polawax NF par la société Croda, la Polawax GP200 vendu par la société Croda.

De préférence, on utilisera, en tant que système émulsionnant un ou plusieurs couples "émulsionnant non ionique de haute HLB" / "émulsionnant non ionique de faible HLB". Il pourra en particulier s'agir d'un système émulsionnant non ionique comprenant au moins un émulsionnant non ionique présentant une HLB supérieure à environ 10 et au moins un émulsionnant non ionique présentant une HLB inférieure à environ 10.

Le ratio de chacun des deux émulsionnants formants le couple précité est déterminé le plus souvent par le calcul de la HLB requise de la phase grasse utilisée.

A titre d'émulsionnant préféré on peut citer :
- des émulsionnants hydrophiles de type PEG 6 stéarate et PEG 32 stéarate vendu sous le nom de TEFOSE 1500 par Gattefossé.
- des émulsionnants lipophiles de type PEG-6 isostéarate vendu sous le nom de Olepal isostéarique® par Gattefossé.

La composition selon l'invention peut également comprendre une phase grasse :
Cette phase grasse peut comprendre par exemple, les huiles végétales, minérales, animales ou synthétiques, des huiles de silicones, et leurs mélanges.

Comme exemple d'huile minérale, on peut citer par exemple des huiles de paraffine de différentes viscosités telles que le Primol 352®, le Marcol 82®, Marcol 152® vendus par la société Esso.

Comme huile végétale, on peut citer l'huile d'amande douce, l'huile de palme, l'huile de soja, l'huile de sésame, l'huile de tournesol, l'huile d'olive.

Comme huile animale, on peut citer la lanoline, le squalene, l'huile de poisson, avec comme dérivé le perhydrosqualene vendu sous le nom Sophiderm® par la société Sophim.

Comme huile synthétique, on peut citer un ester tel que le cetearyl isononanoate comme le produit vendu sous le nom de Cetiol SN PH® par la société Cognis France, le diisopropyl adipate comme le produit vendu sous le nom de Crodamol DA® par la société Croda, le palmitate d'isopropyle comme le produit vendu sous le nom de Crodamol IPP® par la société Croda, le caprylique / caprique triglycéride tel que Miglyol 812® vendu par la société Univar.

Comme huile de silicone, on peut citer une dimethicone comme le produit vendu sous le nom de Q7-9120 Silicone Fluid® de viscosité de 20 cst à 12500 cst par la société Dow Corning, une cyclomethicone comme le produit vendu sous le nom de ST-Cyclomethicone 5NF® également par la société Dow Corning.

On pourra également mettre des corps gras solides tel que des cires naturelles ou synthétiques, des acides gras tels que l'acide stéarique, des alcools gras tels que le Speziol C18 pharma vendu par la société Cognis, et des agents de texture de type tribehenate, tel que le Compritol 888 vendu par la société Gattefossé ou les huiles de ricin hydrogénées telles que le Cutina HR vendu par la société Cognis. Dans ce cas, l'homme du métier adaptera la température de chauffage de la préparation en fonction de la présence ou non de ces solides.

Pour la composition selon l'invention, l'huile de type caprylique caprique triglyceride telle que Miglyol 812®, le cetearyl isononanoate comme le Cetiol SN PH®, et des alcools gras tels que le Speziol C18 pharma sont préférées.

Les compositions de l'invention peuvent comprendre en outre optionnellement tout additif. usuellement utilisé dans le domaine cosmétique ou pharmaceutique tel que des tensioactifs, des agents neutralisants de type bases ou acides usuels, minéraux ou organiques (à titre d'exemple, la triethanolamine, la solution de soude 10%, le tampon acide citrique/sodium citrate, le tampon acide succinique/succinate de sodium), des filtres solaires, des antioxydants (type Butylhydroxyanisole), des charges, des électrolytes, des colorants, ,des parfums, des huiles essentielles, des actifs cosmétiques, des hydratants, des vitamines, des acides gras essentiels, des sphingolipides, des composés autobronzants tels que la DHA, des agents apaisants et protecteurs de la peau tels que l'allantoïne, des agents propénétrants, ou un mélange de ceux-ci. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées.

Ces additifs peuvent être présents dans la composition à raison de 0,0010% à 20 % en poids par rapport au poids total de la composition.

La présente invention a également pour objet la composition telle que décrite précédemment à titre de médicament.

En particulier, l'invention se rapporte à l'utilisation d'une composition telle que décrite précédemment pour la préparation d'un médicament destiné au traitement et/ou à la prévention des affections dermatologiques liées à un désordre de la kératinisation portant sur la différenciation et sur la prolifération cellulaire, notamment pour traiter les acnés vulgaires, comédoniennes, papulopustuleuse, papulocomédoniennes, nodulokystiques, les acnés conglobata, les acnés chéloïdes de la nuque, les acnés miliaires récidivantes, les acnés nécrotiques, les acnés neonatorum, les acnés professionnelles, les acnés rosacées, les acnés séniles, les acnés solaires et les acnés médicamenteuses.

De préférence, l'invention se rapporte à l'utilisation d'une composition telle que décrite précédemment pour la préparation d'un médicament destiné à prévenir et/ou à traiter les acnés vulgaires.

Les compositions selon l'invention sont administrées par voie topique.

En outre, l'invention porte également sur l'utilisation cosmétique d'une composition selon l'invention pour le traitement des peaux à tendance acnéique, pour lutter contre l'aspect gras de la peau ou des cheveux.

Un autre aspect de l'invention se rapporte à un procédé de formulation de complexe comprenant un dérivé d'acide naphtoique et des cyclodextrines et de manière préférée le dérivé d'acide naphtoique est l'adapalene. Le procédé selon l'invention est tel que défini dans la revendication 17.

La présente invention va maintenant être illustrée au moyen des exemples suivants, qui ne sauraient limiter la portée de la présente invention.

### Description des figures :

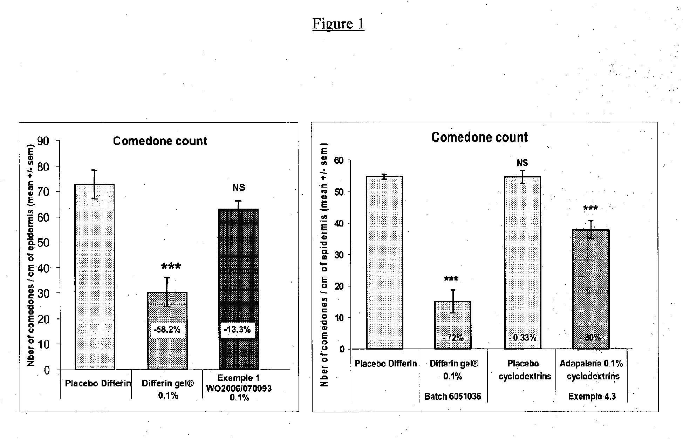

Figure 1 : Evaluation de l'activité comédolytique d'une composition selon l'invention et comparaison à une formulation contenant de l'adapalène solubilisé à 0.1% selon la demande
   WO2006/070093
   NS : non significatif

### EXEMPLES

### Exemple 1 : Procédé de fabrication depuis la formation du complexe jusqu'à l'obtention d'une préparation contenant le complexe Adaplène/cyclodextrine solubilisé.

### 4 étapes :

- Formation du complexe Adapalène / cyclodextrine : Phase 1
- Formation du gel : Phase 2
- Formation de la solution de complexe : Phase 3
- Finalisation : Phase 4

### Phase 1 : Etape de complexation

1) Préparer le mélange de poudre
   a. Adapalène : cyclodextrine RAMEB (1: n), n étant le nombre de moles de cyclodextrine
   b. Ajout de 10% d'eau
2) Mettre sous agitation
3) Laisser à maturation 2 heures à 60°C et 150 bars
4) Phase de séchage : 50°C sous vide durant une nuit

### Phase 2 : Formation de la solution de complexe

1) Peser et introduire l'eau purifiée dans le flacon
2) Peser et introduire la cyclodextrine libre RAMEB, maintenir l'agitation jusqu'à solubilisation
3) peser le complexe Adapalène/cyclodextrine
4) Introduire le complexe dans la solution de cyclodextrine libre RAMEB
5) Mettre sous agitation afin d'homogénéiser le mélange
6) Une fois homogénéisé, filtrer le mélange avec un filtre de diamètre compris entre 0.2 et 1µm. La filtration est correcte seulement dans le cas ou le mélange obtenu est limpide
7) La solution de complexe ainsi obtenue doit contenir 0.2% (m/m) ou 0.6% (m/m) d'Adapalène

### Phase 3 : Formation de la matrice formulaire

### Etape a : Préparation de la phase aqueuse

Dans un bêcher, on introduit sous agitation, si nécessaire à chaud, de l'eau purifiée et, le ou les conservateurs, et optionnellement le ou les agents gélifiants, le ou les agents chélatants, le ou les émulsionnants hydrophiles, le ou les agents stabilisants, le ou les humectants et/ou émollients.

Le mélange est porté à 80°C environ.

### Etape b :

Introduire dans le bêcher formulaire précédent, la cyclodextrine libre RAMEB en excès, et maintenir l'agitation jusqu'à solubilisation.

### Etape c (optionnellement pour une émulsion) :

Ajouter la quantité nécessaire de solution de complexe obtenue lors de la phase 2 du présent mode opératoire dans la phase aqueuse précédente (obtenue à l'étape b).

### Etape d (optionnellement pour l'obtention d'une émulsion): Préparation de la phase grasse :

Mélange des émulsionnants lipophiles, des composés huileux, des corps gras solides et optionnellement des émulsionnants lipophiles, des conservateurs. Le mélange est porté à environ 75°C.

Le mélange est chauffé et après homogénéisation, le silicone volatile est introduit en dernier si présent dans la composition.

### Etape e (optionnelle): Emulsification :

A la température de 75°C, la phase grasse (étape d) est introduite dans la phase aqueuse (étape c) sous agitation afin de réaliser l'émulsification.

### Etape f : Addition de l'agent gélifiant (pour la préparation d'un gel)

On introduit sous agitation le ou les agents gélifiants à la phase obtenue en b) pour le gel. L'agitation est maintenue jusqu'à parfaite homogénéité.

### Etape g : Neutralisation :

L'agent de neutralisation du gélifiant est introduit si nécessaire, Dans le cas d'un gel, il sera introduit dans la phase obtenue à l'étape f). Pour une émulsion, il sera introduit dans la phase obtenue à l'étape e).

### Etape h : (optionnelle) ajustement en eau

Si nécessaire, un ajustement en eau est réalisé.

### Etape i (pour la préparation d'un gel à 0.1% ou à 0.3% en adapalène) : Addition de la solution de complexe

Après dispersion et neutralisation si nécessaire de l'agent gélifiant, ajouter en quantités égales la préparation obtenue à l'étape h) et la solution de complexe réalisée lors de la phase 2. Par exemple, pour la préparation de 100 g après dispersion et neutralisation si nécessaire de l'agent gélifiant, ajouter à 50 g de préparation obtenue à l'étape h), 50 g de solution de complexe réalisée lors de la phase 2 du présent mode opératoire.
Mettre sous agitation jusqu'à homogénéisation.

Cette invention permet la formulation de compositions contenant 0.1% m/m et 0.3% m/m d'Adapalène sous forme solubilisée grâce à l'utilisation de cyclodextrines.

### Exemple 2 :

### 2.1. Mise en solution du complexe Adapalène/cyclodextrine

Le travail a été effectué à partir de trois complexes caractérisés par 3 ratios molaires Adapalene/cyclodextrine différents, ces trois ratios ont été obtenus au cours de la fabrication du complexe par SCF :
- Complexe 1 : 1/6
- Complexe 2 : 1/8
- Complexe 3 : 1/10

Le tableau ci-dessous récapitule les caractéristiques de ces 3 complexes:

**Tableau 1 : Présentation des 3 complexes étudiés**

| | Ratio molaire Adapalène/Cyclodextrine | % d'Adapalène présent en théorie dans le complexe | % d'Adapalène présent dans le complexe suite aux dosages analytiques réalisés |
|---|---|---|---|
| Complexe 1 | 1/6 | 4.8 | 4.709 |
| Complexe 2 | 1/8 | 3.1 | 3.832 |
| Complexe 3 | 1/10 | 2.8 | 3.082 |

La cyclodextrine présente dans le complexe est la RAMEB (Randomly Methylated β-cyclodextrine).

Il a été cherché à solubiliser les complexes Adapalène/cyclodextrine définis ci-dessus dans l'eau purifiée.

Les concentrations en Adapalène que nous souhaitons obtenir sont de 0.2% et 0.6% (m/m) en solution dans l'eau. Ces concentrations ont été choisies dans l'objectif de diluer ces solutions de complexe, une fois stabilisées, dans une forme galénique pour obtenir par exemple; une forme gel à 0.1 ou 0.3% (m/m) d'Adapalène.

### 2.2 Mode opératoire pour obtenir une solution stable de complexe Adapalène/Cyclodextrine

Les premiers essais de mise en solution des complexes, montrent que les mélanges obtenus sont laiteux, la totalité du complexe Adapalène/cyclodextrine introduit dans l'eau purifiée ne se solubilise pas. Nous avons filtré ce premier mélange pour obtenir une solution limpide de complexe Adapalène/cyclodextrine.
Les premiers essais de stabilité des solutions de complexe ont montré une décomplexation rapide de l'Adapalène dans l'eau purifiée.
Afin de modifier ou de ralentir la cinétique de décomplexation, nous avons choisi d'ajouter de la cyclodextrine libre dans l'eau purifiée avant de solubiliser le complexe Adapalène/cyclodextrine.

Le mode opératoire utilisé pour la réalisation de la solution de complexe est le suivant :
▪ Dans un flacon, peser l'eau purifiée
▪ Additionner si nécessaire la cyclodextrine libre (Cavasol W7M Pharma)
▪ Mettre sous agitation magnétique afin d'homogénéiser le mélange
- Une fois le mélange homogénéisé, peser le complexe Adapalène/cyclodextrine et l'introduire dans le flacon
▪ Mettre sous agitation afin d'homogénéiser le mélange environ 2h (il ne doit plus y avoir de grumeaux dans le flacon ; la solution est laiteuse)
▪ Une fois homogénéisé, filtrer le mélange sur un filtre de diamètre compris entre 0,2 et 1 µm. La filtration est correcte seulement dans le cas ou le mélange obtenu est limpide
▪ La solution de complexe ainsi obtenue doit contenir 0.2% (m/m) ou 0.6% (m/m) d'Adapalène

Comme mentionnée précédemment, une étape de filtration est nécessaire pour obtenir une solution limpide de complexe Adapalène/Cyclodextrine.
Une fois filtrées, les solutions de complexe sont dosées afin de déterminer leur titre exact en Adapalène. Ce titre permet de déterminer la perte en Adapalène au cours de l'étape de filtration. Cet essai a été réalisé plusieurs fois, et la perte en Adaplène est reproductible. Connaissant la valeur de la perte en Adapalène lors de la filtration, la quantité exacte de complexe à incorporer initialement au cours de la réalisation de la solution peut être déterminée.

### 2.3 Analyses chimiques des solutions de complexes adapalène/Cyclodextrine

Des dosages analytiques ont été réalisés afin de déterminer le titre en Adapalène après filtration de la solution de complexe. Ceci de manière à connaître la quantité exacte de complexe à incorporer initialement dans l'eau purifiée en fonction du complexe mis en oeuvre.

Le tableau ci-dessous présente les pertes en Adapalène en fonction du ratio Adapalène/cyclodextrine mis en oeuvre dans le complexe :

| Complexe Adapalène/M-Beta cyclodextrine | Concentration et perte en Adapalène | Solution de complexe sans cyclodextrine libre | Solution de complexe avec cyclodextrine libre (80g/l) |
|---|---|---|---|
| Complexe 1 (1/6) | Concentration initiale en Adapalène (mg/g) | 4.995 | 5.080 |
| | Concentration finale en Adapalène (mg/g) | 3.935 | 4.008 |
| | **Perte en Adapalène (%)** | **21** | **21** |
| Complexe 2 (1/8) | Concentration initiale en Adapalène (mg/g) | 3.238 | 3.395 |
| | Concentration finale en Adapalène (mg/g) | 2.910 | 3.140 |
| | **Perte en Adapalène (%)** | **10.1** | **7.5** |
| Complexe 3 (1/10) | Concentration initiale en Adapalène (mg/g) | 4.999 | 5.000 |
| | Concentration finale en Adapalène (mg/g) | 4.415 | 4.291 |
| | **Perte en Adapalène (%)** | **11.7** | **14.2** |

Conclusion : les pertes en Adapalène sont reproductibles en fonction du complexe mis en oeuvre et ne semblent pas dépendre significativement de l'ajout ou non de cyclodextrine libre. Pour la suite des essais, la pesée en complexe Adaplène/Cyclodextrine tiendra compte de cette perte en Adapalène qui a lieu au cours de la filtration.

Dans le cas du complexe 3, la perte en adapalène est d'environ 15%. Pour ce complexe, le ratio est de 1 pour 10. On peut donc estimer la quantité maximum de cyclodextrine libre dans la solution, après filtration, liée à la décomplexation au cours de la solubilisation.
Au maximum, on libère environ 7 mg/g de cyclodextrine, on aura donc au maximum 87g/l de cyclodextrine libre dans la solution intermédiaire pour le complexe 3 (1/10).

### 2.4 Stabilité physique des solutions des complexes Adapalène/Cyclodextrine

La stabilité physique du complexe se caractérise visuellement, les systèmes restent limpides tant que le complexe est intègre. Dès que l'Adapalène est décomplexé, le système se trouble du fait de la précipitation de l'Adapalène dans la solution étudiée.

Données de stabilité physique des solutions de complexe :

| | **Conditions de stabilité** | |
|---|---|---|
| | **Température ambiante (TA)** | **4°C** |
| Complexe 1 (1/6) + eau purifiée | Présence de cristaux d'Adapalène dès T_{+96H} | Présence de cristaux d'Adapalène dès T_{+96H} |
| Complexe 1 (1/6) + RAMEB (80g/l) + eau purifiée | Limpide à T_{+3 mois} | Limpide à T_{+3 mois} |
| Complexe 2 (1/8) + eau purifiée | Présence de cristaux d'Adapalène dès T_{+24H} | |
| Complexe 2 (1/8) + RAMEB (80g/l) + eau purifiée | Limpide à T_{+2,5 mois} | Limpide à T_{+2,5 mois} |
| Complexe 3 (1/10) + eau purifiée | Limpide à T_{+1 mois st demi} | Limpide à T_{+1 mois et demi} |
| Complexe 3 (1/10) + RAMEB (80g/l) + eau purifiée | Limpide à T_{+2.5 mois} | Limpide à T_{+2.5 mois} |

Le complexe de Adapalène/cyclodextrine en solution est stable à 4°C, et température ambiante pendant au moins 2,5 mois, lorsque de la cyclodextrine libre est présente en excès dans la solution.

### Conclusions :

D'après les résultats ci-dessus, lorsque les complexes Adapalène/cyclodextrine sont solubilisés dans l'eau purifiée sans cyclodextrine libre, la stabilité du complexe est nettement améliorée dans le cas où le ratio molaire est le plus faible : Complexe 3 - 1/10 (2.8% d'adapalène). Cette solution est stable au moins 2 mois et demi, quelles que soient les conditions de mise en température.

L'ajout de cyclodextrine libre dans l'eau, dans le but de modifier la cinétique de décomplexation du complexe l'Adapalène/Cyclodextrine, améliore significativement la stabilité des complexes solubilisés. Pour le complexe 1 (1/6: 4.8% d'adapalène), les solutions sont stables jusqu'à 3 mois à température ambiante en comparaison avec les solutions sans cyclodextrine libre où le même complexe se déstabilisait au bout de 96h.

Pour réaliser les formulations gélifiées à 0.1 et 0.3% d'adapalène, il faut tenir compte des pertes en Adapalene définies précédemment, lors de la mise en solution du complexe. Le tableau ci-dessous présente les titres en adapalène des solutions de complexe mises en oeuvre pour la suite de l'invention :

| Nature de la solution de complexe | Conditions de stabilité |
|---|---|
| | Température ambiante |
| Complexe 3 (0.6% Adapalène) / RAMEB 100g/L/ eau purifiée | 6.156 mg/g (103) |
| Complexe 3 (0.2% Adapalène) / RAMEB 100g/L/ eau purifiée | 2.103 mg/g (105) |

### 2.5 Mise en oeuvre de matrice formulaire contenant le complexe Adapalène/Cyclodextrine solubilisé

Les essais de stabilisation du complexe Cyclodextrine/Adapalène en solution, montrent qu'il est nécessaire d'introduire 80 à 100 g/l de cyclodextrine libre dans le milieu de solubilisation du complexe pour le stabiliser.

Les premiers essais de formulation dans une base gel des solutions de complexes contenant des cyclodextrines libres ont déstabilisé le complexe.
En effet, différents essais ont montré que si la base gel ne contenait pas de cyclodextrine libre, l'introduction de la solution de complexe dans cette base génère la décomplexation de l'Adapalène très rapidement dans la formulation.

Il est donc nécessaire de former une matrice formulaire présentant la même concentration en cyclodextrine libre que celle mise en oeuvre dans la solution de complexe.

### Exemple 3 : Procédé de fabrication des formes gélifiées

### Phase 1 : Formation de la solution intermédiaire de complexe

1) Peser et introduire l'eau purifiée dans le flacon
2) Peser et introduire la cyclodextrine libre RAMEB, maintenir l'agitation jusqu'à solubilisation
3) peser le complexe Adapalène/cyclodextrine
4) Introduire le complexe dans la solution de cyclodextrine libre RAMEB
5) Mettre sous agitation afin d'homogénéiser le mélange
6) Une fois homogénéisé, filtrer le mélange avec un filtre de diamètre moyen compris entre 0.2 et 1µm. La filtration est correcte seulement dans le cas ou le mélange obtenu est limpide
7) La solution intermédiaire de complexe ainsi obtenue doit contenir 0.2% (m/m) ou 0.6% (m/m) d'Adapalène

### Phase 2 : Formation du gel

1) Dans un bécher, peser l'eau purifiée, mettre sous agitation et chauffer jusqu'à 80°C à l'aide d'une plaque chauffante
2) Peser et introduire le Méthyl Paraben
3) Laisser sous agitation jusqu'à totale dissolution du Méthyl Paraben
4) Laisser refroidir la solution
5) Peser et introduire la cyclodextrine libre RAMEB, maintenir l'agitation jusqu'à solubilisation
6) peser et introduire le Propane-diol 1,2, l'EDTA et le phénoxyéthanol
7) Homogénéiser le mélange
8) Peser et introduire le gélifiant aqueux
9) Lorsque le gélifiant est parfaitement dispersé, neutraliser avec la solution de soude 10% (seulement si le gélifiant est le Carbopol 980NF)
10) Laisser sous agitation jusqu'à homogénéisation
11) Faire un qsp en eau si nécessaire afin de compenser les pertes en eau suite à l'évaporation

### Phase 3 : Finalisation (pour une préparation de 100g et obtention d'une formule contenant 0.1% (m/m) ou 0.3% (m/m) d'Adapalène)

1) Réaliser une prise d'essai de 50g de gel (obtenu à l'étape 2) et l'introduire dans un bécher
2) Mettre sous agitation à 200 tr/min
3) Peser et introduire dans le bécher 50g de la solution de complexe réalisée à l'étape 1
4) Laisser sous agitation jusqu'à homogénéisation

### Exemple 4 : Formulations comprenant de l'adapalène à 0,1% et 0,3%

La présente invention va maintenant être illustrée au moyen des exemples suivants et des données de stabilités physique et chimique présentées ci-dessous.

### Exemple 4.1 : Gel Adapalène/Cyclodextrine à 0.1% d'Adapalène

| **Matières Premières** | **Teneur (% m/m)** |
|---|---|
| Carbopol 980NF | 1.1 |
| Solution de soude 10% | 1.6 |
| Complexe 1 Adapalène/Cyclodextrine (1/6) | 2.083 |
| RAMEB | 8.0 |
| Eau purifiée | Qsp 100 |

### Exemple 4.2 : Gel Adapalène/Cyclodextrine à 0.1% d'Adapalène

| **Matières premières** | **Teneur (% m/m)** |
|---|---|
| Carbopol 980NF | 1.1 |
| Solution de soude 10% | 1.6 |
| Complexe 3 Adapalène/Cyclodextrine (1/10) | 3.571 |
| RAMEB | 8.0 |
| Eau purifiée | Qsp 100 |

### Exemple 4.3: Gel Adapalène/Cyclodextrine à 0.1% d'Adapalène

| **Matières premières** | **Teneur (% m/m)** |
|---|---|
| Carbopol 980NF | 1.1 |
| Propane-diol 1,2 | 4.0 |
| EDTA | 0.1 |
| Méthyl Parabène | 0.1 |
| Phénoxyéthanol | 0.25 |
| Solution de soude 10% | 1.6 |
| Complexe 3 Adapalène/Cyclodextrine (1/10) | 3.571 |
| RAMEB | 10.0 |
| Eau purifiée | Qsp 100% |

### Exemple 4.4 : Gel Adapalène/Cyclodextrine à 0.3% d'Adapalène

| **Matières premières** | **Teneur (% m/m)** |
|---|---|
| Carbopol 980NF | 1.1 |
| Propane-diol 1,2 | 4.0 |
| EDTA | 0.1 |
| Méthyl Parabène | 0.1 |
| Phénoxyéthanol | 0.25 |
| Solution de soude 10% | 1.6 |
| Complexe 3 Adapalène/Cyclodextrine (1/10) | 10.714 |
| RAMEB | 10.0 |
| Eau purifiée | Qsp 100% |

### Exemple 4.5 : Gel Adapalène/Cyclodextrine à 0.3% d'Adapalène

| **Matières premières** | **Teneur (% m/m)** |
|---|---|
| Simulgel 600PHA | 2 |
| Propane-diol 1,2 | 2.0 |
| EDTA | 0.1 |
| Docusate de Sodium | 0.05 |
| Glycérie | 2 |
| Complexe 3 Adapaléne/Cyclodextrine (1/10) | 10.714 |
| RAMEB | 10.0 |
| Solution de soude 10% (m/m) | Qsp pour pH 4,5 |
| Eau purifiée | Qsp 100% |

### Exemple 4.6 : Gel-crème Adapalène/Cyclodextrine à 0.3 % d'Adapalène

| **Matières premières** | **Teneur (% m/m)** |
|---|---|
| Carbopol Ultrez 20 | 0.35 |
| Gomme Xanthane | 0.1 |
| Caprylique / Caprique Triglycédrides | 7 |
| Propane-diol 1,2 | 3.0 |
| EDTA | 0.1 |
| Glycérine | 3 |
| Complexe 3 Adapalène/Cyclodextrine (1/90) | 10.714 |
| RAMEB | 10.0 |
| Eau purifiée | Qsp 100% |

### Exemple 4.7 : Gel-crème Adapalène/Cyclodextrine à 0.3 % d'Adapalène

| **Matières premières** | **Teneur (% m/m)** |
|---|---|
| Carbopol 1382 | 0.35 |
| Hydroxypropylmethylcellulose | 0.1 |
| Squalane | 4 |
| PEG-6 Isostearate | 1 |
| Cetearyl isononanoate | 4 |
| Propyl Paraben | 0.05 |
| Lauroglycol | 2.0 |
| EDTA | 0.1 |
| Complexe 3 Adapalène/Cyclodextrine (1/10) | 10.714 |
| RAMEB | 10.0 |
| Solution de soude 10% (m/m) | Qsp pour pH 4,5 |
| Eau purifiée | Qsp 100% |

### Exemple 4.8 : Lotion Adapalène/Cyclodextrine à 0.3% d'Adapalène

| **Matières premières** | **Teneur (% m/m)** |
|---|---|
| Carbopol ultrez 20 | 0.15 |
| Propylène glycol | 2 |
| HEDTA | 0.1 |
| Méthyl Parabène | 0.1 |
| Alcool stéarylique | 2 |
| Propyl Parabène | 0.05 |
| PEG-6 stéarate / PEG-32 stéarate | 2.5 |
| Caprylique / Caprique Triglycédrides | 3.5 |
| Complexe 3 Adapalène/Cyclodextrine (1/10) | 10.714 |
| RAMEB | 10.0 |
| Solution de soude 10% (m/m) | Qsp pour pH 4,5 |
| Eau purifiée | Qsp 100% |

### Exemple 5 : Stabilité physique des formulations

Comme pour les solutions de complexe présentées ci-dessus, la caractérisation de la stabilité du complexe Adapalène/Cyclodextrine dans les formulations gélifiées se fait visuellement. La limpidité de la formulation justifie de la stabilité du complexe.

### Caractérisations à T0

| **T0** | **Exemple 4.3** | **Exemple 4.4** |
|---|---|---|
| **Aspect macroscopique** | Gel épais transparent incolore | Gel épais transparent incolore |
| **Microscopie Zeiss x40** | Absence de cristaux. Fluorescence diffuse | Absence de cristaux. Fluorescence diffuse |
| **pH** | 4.85 | 4.85 |

### Stabilité 6 mois à +4°C, TA et 40°C

| **T 6 mois** | | **Exemple 4.3** | **Exemple 4.4** |
|---|---|---|---|
| **Aspect macroscopique** | | Conforme à T0 à toutes les températures | Conforme à T0 à toutes les températures |
| **Microscopie Zeiss x40** | | Conforme à T0 à toutes les températures | Conforme à T0 à toutes les températures |
| **pH** | **TA** | 5.02 | 5.06 |
| | **+4°C** | 5.02 | 5.73 |
| | **+40°C** | 4.99 | 5.06 |

### Exemple 6 : Stabilité chimique des formulations

### Caractérisations à T0

| **T0** | **Exemple 4.3** | **Exemple 4.4** |
|---|---|---|
| **Dosage de l'adapalène (% du titre théorique)** | 105 | 101 |

### Stabilité 6 mois à +4°C, TA et 40°C

| **T6 mois** | | **Exemple 4.3** | **Exemple 4.4** |
|---|---|---|---|
| **Dosage de l'adapalène (% du titre théorique)** | **TA** | 102 | 107 |
| | **+4°C** | 103 | 106 |
| | **+4°C** | 107 | 107 |

Les formulations avec 0.1 et 0.3% d'adapalene complexé, contenant de la cyclodextrine libre sont stables physiquement et chimiquement pendant au moins 6 mois aux trois conditions de température testées (+4°C, TA et 40°C).

### Exemple 6: Etude in vitro des propriétés de libération-pénétration des préparations d'adapalène solubilisé

La composition de l'exemple 4.3 est comparée dans un test de libération/pénétration connu de l'homme du métier afin d'évaluer la quantité d'actif pénétré.

Les résultats expérimentaux (tableau ci-dessous) montrent que quelle que soit la formulation testée, l'adapalène est distribué principalement dans la peau (épiderme, *stratum corneum* inclus, et derme). Les quantités totales pénétrées sont :

| Formulations contenant de | | Épiderme (+ *Stratum* | Derme | Dose absorbée dans le liquide | Quantité totale |
|---|---|---|---|---|---|
| l'Adapalene | | *corneum*) | | récepteur | pénétrée |
| | | | | | |

| **Référence : Differin Ò Gel** | | | | | |
|---|---|---|---|---|---|
| 0.1% | µg | 0.16 ± 0,04 | 0.01 ± 0.01 | <LQ | 0.17 ± 0.05 |
| | % | *1.77* ± *0.42* | *0.10* ± *0.05* | | *1.88* ± *0.45* |

| **Cyclodextrine gels** | | | | | |
|---|---|---|---|---|---|
| Adapalène 0.1% | µg | 1.28 ± 0.33 | 0.10 ± 0.03 | <LQ | 1.38 ± 0.36 |
| Exemple 4.3 | % | *14.44* ± *3.94* | *1.11* ± *0.33* | | *15*.*55* ± *4*.*22* |

| | | | | | |
|---|---|---|---|---|---|
| <LQ : Inférieur à la limite de quantification | | | | | |

La quantité totale d'adapalène ayant pénétré est de 1,88% de la dose appliquée pour Différine® gel 0.1% (m/m) et de 15,55% de la dose appliquée pour lé préparation selon l'exemple 4.3 de la présente invention.

Ces résultats montrent de manière significative, une meilleure libération et une meilleure pénétration in vitro sur peau humaine, de l'adapalène solubilisé selon la présente invention, par rapport au produit de référence Différine® gel. L'adapalène complexé et solubilisé pénètre de manière significative 8 fois plus dans la peau que le produit de référence.

### Exemple 7 : Evaluation de l'activité comédolytique d'une composition selon l'invention

La composition de l'exemple 4.3 est comparée dans un test de mesure de l'activité comédolytique à un gel d'adapalène dispersé (Différine® gel à 0.1%) et à une préparation contenant 0.1% d'adapalène solubilisé en milieu aqueux avec des cyclodextrines, décrite selon l'exemple 1 de la demande WO2006/070093 (à savoir un mélange physique d'adapalene et de cyclodextrines sans la technologie permettant de bénéficier du pouvoir de solvant d'un fluide dense sous pression).

### Résultant:

La figure 1 montre qu'aux mêmes concentrations d'actif, la composition selon l'exemple 4.3 de la présente invention contenant de l'adaplène solubilisé, montre une activité comédolytique supérieure à la composition dans laquelle adapalène est solubilisé et formulé selon l'exemple 1 de la demande WO2006/070093.

En effet, la différence observée entre l'exemple 1 de la demande WO2006/070093 et le placebo gel de Différine® est non significative (t-test) alors que l'on note une diminution de 30% du nombre de comédons avec la formule préparée selon l'exemple 4.3 de la présente invention par rapport au placebo.

## Revendications

1. Composition topique comprenant, dans un milieu physiologiquement acceptable, au moins un complexe composé d'un dérivé d'acide naphtoïque de formule (I), leurs sels et esters : 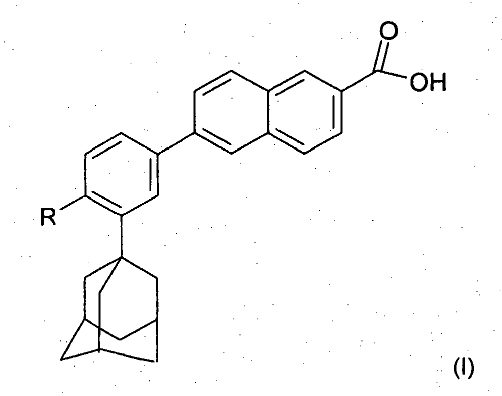 où R représente un atome d'hydrogène, un radical hydroxyle, un radical alkyle, ramifié ou non, ayant de 1 à 4 atomes de carbone, un radical alkoxy ayant de 1 à 10 atomes de carbone ou un radical cycloaliphatique substitué ou non, dont la concentration est comprise entre 0,001% et 10%, préférentiellement entre 0,01% et 5% et, plus préférentiellement, entre 0,05% et 2% en poids du poids total de la composition,
et d'au moins une méthyl-β-cyclodextrine ledit complexe moléculaire soluble est obtenu par la technologie de fluides denses sous pression.

2. Composition selon la revendication 1, **caractérisée en ce que** le fluide dense sous pression est du CO2 supercritique.

3. Composition selon la revendication 1, **caractérisée en ce que** le radical alkyle dans la formule (I) est le radical méthyle, éthyle, propyle ou butyle.

4. Composition selon la revendication 1 ou 3, **caractérisée en ce que** le radical alkoxy dans la formule (I) est le radical méthoxy, éthoxy, propoxy, butoxy, hexyloxy ou décyloxy.

5. Composition selon l'une des revendications 1 à 4, **caractérisée en ce que** le radical cycloaliphatique dans la formule (I) est le radical methyl-1 cyclohexyle ou le radical 1-adamantyle.

6. Composition selon l'une des revendications 1 à 5, **caractérisée en ce que** le composé dérivé de l'acide naphtoïque est choisi parmi l'adapalène, l'acide 6-[3-(1-adamantyl)-4-hydroxyphenyl]-2-naphtoïque, l'acide 6-[3-(1-adamantyl)-4-decyloxyphenyl]-2-naphtoïque et l'acide 6-[3-(1-adamantyl)-4-hexyloxyphenyl]-2-naphtoïque.

7. Composition selon la revendication 6, **caractérisée en ce que** le composé dérivé d'acide naphtoïque est l'adapalène.

8. Composition selon la revendication 1, **caractérisée en ce que** la méthyle-β-cyclodextrine est choisie parmi les TRIMEB (heptakis (2,3,6-trimethyl)-β-CD), les DIMEB (heptakis (2,6-dimethyl)-β-CD) ou encore les RAMEB (Randomly Methylated β-Cyclodextrine).

9. Composition selon la revendication 8, **caractérisée en ce que** la méthyl-β-cyclodextrine est celle de type RAMEB (Randomly Methylated β-Cyclodextrine).

10. Composition selon l'une des revendications 1 à 9, **caractérisée en ce qu'**elle se présente sous la forme d'un gel.

11. Composition selon les revendications 1 à 10, **caractérisée en ce que** la concentration en composé dérivé d'acide naphtoïque est égale à 0,1%.

12. Composition selon la revendications 11, **caractérisée en ce que** la concentration en composé dérivé d'acide naphtoïque est égale à 0,3%.

13. Composition selon l'une quelconque des revendications précédentes à titre de médicament.

14. Utilisation d'une composition selon l'une des revendications 1 à 12 pour la préparation d'un médicament destiné au traitement et/ou à la prévention des affections dermatologiques liées à un désordre de la kératinisation portant sur la différenciation et sur la prolifération cellulaire notamment pour traiter les acnés vulgaires, comédoniennes, papulopustuleuse, papulocomédoniennes, nodulokystiques, les acnés conglobata, les acnés chéloïdes de la nuque, les acnés miliaires récidivantes, les acnés nécrotiques, les acnés neonatorum, les acnés professionnelles, les acnés rosacées, les acnés séniles, les acnés solaires et les acnés médicamenteuses.

15. Utilisation d'une composition selon l'une des revendications 1 à 12 pour la préparation d'une composition pharmaceutique destinée à prévenir et/ou à traiter les acnés vulgaires.

16. Utilisation cosmétique d'une composition selon l'une quelconque des revendications 1 à 12 pour le traitement des peaux à tendance acnéique ou pour lutter contre l'aspect gras de la peau ou des cheveux.

17. Procédé de formulation d'une composition comprenant un complexe composé d'un dérivé d'acide naphtoic et de cyclodextrine et de préférence d'Adaplène et de /cyclodextrine, **caractérisé en ce qu'**il comprend les étapes suivantes :
Phase 1 : Etape de complexation
1) Préparer le mélange de poudre de
a. Dérivé d'acide naphtoique, préférentiellement l'Adapalène: cyclodextrine RAMEB (1 : n), n étant le nombre de moles de cyclodextrine
b. Ajout de 10% d'eau
2) Mettre sous agitation
3) Laisser à maturation 2 heures à 60°C et 150 bars
4) Phase de séchage : 50°C sous vide durant une nuit
Phase 2 : Formation de la solution de complexe
1) Peser et introduire l'eau purifiée dans le flacon
2) Peser et introduire la cyclodextrine libre RAMEB, maintenir l'agitation jusqu'à solubilisation
3) peser le complexe Adapalène/cyclodextrine
4) Introduire le complexe dans la solution de cyclodextrine libre RAMEB
5) Mettre sous agitation afin d'homogénéiser le mélange
6) Une fois homogénéisé, filtrer le mélange avec un filtre de diamètre compris entre 0.2 et 1µm. La filtration est correcte seulement dans le cas ou le mélange obtenu est limpide
7) La solution de complexe ainsi obtenue doit contenir 0.2% (m/m) ou 0.6% (m/m) d'Adapalène
Phase 3 : Formation de la matrice formulaire
Etape a : Préparation de la phase aqueuse
Dans un bécher, on introduit sous agitation, si nécessaire à chaud, de l'eau purifiée et, le ou les conservateurs, et optionnellement le ou les agents gélifiants, le ou les agents chélatants, le ou les émulsionnants hydrophiles, le ou les agents stabilisants, le ou les humectants et/ou émollients.
Le mélange est porté à 80°C environ.
Etape b :
Introduire dans le bécher formulaire précédent, la cyclodextrine libre RAMEB en excès, et maintenir l'agitation jusqu'à solubilisation.
Etape c (optionnellement pour une émulsion) :
Ajouter la quantité nécessaire de solution de complexe obtenue lors de la phase 2 du présent mode opératoire dans la phase aqueuse précédente (obtenue à l'étape b).
Etape d (optionnellement_pour l'obtention d'une émulsion): Préparation de la phase grasse :
Mélange des émulsionnants lipophiles, des composés huileux, des corps gras solides et optionnellement des émulsionnants lipophiles, des conservateurs. Le mélange est porté à environ 75°C.
Le mélange est chauffé et après homogénéisation, le silicone volatile est introduit en dernier si présent dans la composition.
Etape e (optionnelle): Emulsification :
A la température de 75°C, la phase grasse (étape d) est introduite dans la phase aqueuse (étape c) sous agitation afin de réaliser l'émulsification.
Etape f : Addition de l'agent gélifiant (pour la préparation d'un gel)
On introduit sous agitation le ou les agents gélifiants à la phase obtenue en b) pour le gel. L'agitation est maintenue jusqu'à parfaite homogénéité.
Etape g : Neutralisation :
L'agent de neutralisation du gélifiant est introduit si nécessaire, Dans le cas d'un gel, il sera introduit dans la phase obtenue à l'étape f). Pour une émulsion, il sera introduit dans la phase obtenue à l'étape e).
Etape h : (optionnelle) ajustement en eau
Si nécessaire, un ajustement en eau est réalisé.
Etape i (pour la préparation d'un gel à 0.1% ou à 0.3% en adapalène) : Addition de la solution de complexe
Phase 4 : finalisation
Après dispersion et neutralisation si nécessaire de l'agent gélifiant, ajouter en quantités égales la préparation obtenue à l'étape h) et la solution de complexe réalisée lors de la phase 2. Mettre sous agitation jusqu'à homogénéisation.

## Patentansprüche

1. Topische Zusammensetzung, die in einem physiologisch unbedenklichen Medium mindestens einen Komplex umfasst, der sich aus einem Naphthoesäurederivat der Formel (I), Salzen und Estern davon: 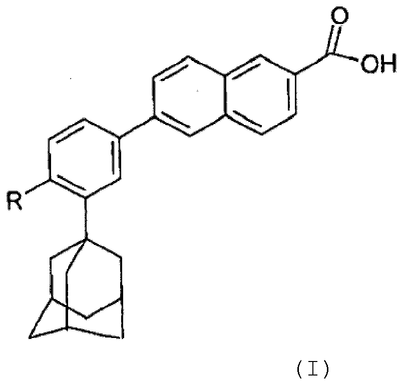 wobei R für ein Wasserstoffatom, einen Hydroxylrest, einen verzweigten oder unverzweigten Alkylrest mit 1 bis 4 Kohlenstoffatomen, einen Alkoxyrest mit 1 bis 10 Kohlenstoffatomen oder einen substituierten oder unsubstituierten cycloaliphatischen Rest steht, dessen Konzentration zwischen 0,001 und 10 Gew.-%, vorzugsweige zwischen 0,01 und 5 Gew.-% und stärker bevorzugt zwischen 0,05 und 2 Gew.-% des Gesamtgewichts der Zusammensetzung liegt,
und aus mindestens einem Methyl-β-cyclodextrin zusammensetzt, wobei der lösliche molekulare Komplex mittels der Technik der dichten, unter Druck stehenden Fluids hergestellt wird.

2. Zusammensetzung nach Anspruch 1, **dadurch gekennzeichnet, dass** es sich bei dem dichten, unter Druck stehenden Fluid um überkritisches CO2 handelt.

3. Zusammensetzung nach Anspruch 1, **dadurch gekennzeichnet, dass** es sich bei dem Alkylrest in der Formel (I) um den Methyl-, Ethyl-, Propyl- oder Butylrest handelt.

4. Zusammensetzung nach Anspruch 1 oder 3, **dadurch gekennzeichnet, dass** es sich bei dem Alkoxyrest in der Formel (I) um den Methoxy-, Ethoxy-, Propoxy-, Butoxy-, Hexyloxy- oder Decyloxyrest handelt.

5. Zusammensetzung nach einem der Ansprüche 1 bis 4, **dadurch gekennzeichnet, dass** es sich bei dem cycloaliphatischen Rest in der Formel (I) um den Methyl-1-cyclohexylrest oder den 1-Adamantylrest handelt.

6. Zusammensetzung nach einem der Ansprüche 1 bis 5, **dadurch gekennzeichnet, dass** die Verbindung, welche sich von Naphthoesäure ableitet, aus Adapalen, 6-[3-(1-Adamantyl)-4-hydroxyphenyl]-2-naphthoesäure, 6-[3-(1-Adamantyl)-4-decyloxyphenyl]-2-naphthoesäure und 6-[3-(1-Adamantyl)-4-hexyloxyphenyl]-2-naphthoesäure ausgewählt ist.

7. Zusammensetzung nach Anspruch 6, **dadurch gekennzeichnet, dass** es sich bei der Verbindung, welche sich von Naphthoesäure ableitet, um Adapalen handelt.

8. Zusammensetzung nach Anspruch 1, **dadurch gekennzeichnet, dass** das Methyl-β-cyclodextrin aus den TRIMEB (Heptakis-(2,3,6-trimethyl)-β-CD), den DIMEB (Heptakis-(2,6-dimethyl)-β-CD) oder auch den RAMEB (Randomly Methylated β-Cyclodextrine) ausgewählt ist.

9. Zusammensetzung nach Anspruch 8, **dadurch gekennzeichnet, dass** es sich bei dem Methyl-β-cyclodextrin um dasjenige vom Typ RAMEB (Randomly Methylated β-Cyclodextrine) handelt.

10. Zusammensetzung nach einem der Ansprüche 1 bis 9, **dadurch gekennzeichnet, dass** sie in Form eines Gels vorliegt.

11. Zusammensetzung nach den Ansprüchen 1 bis 10, **dadurch gekennzeichnet, dass** die Konzentration der Verbindung, welche sich von Naphthoesäure ableitet, gleich 0,1 % ist.

12. Zusammensetzung nach Anspruch 11, **dadurch gekennzeichnet, dass** die Konzentration der Verbindung, welche sich von Naphthoesäure ableitet, gleich 0,3 % ist.

13. Zusammensetzung nach einem beliebigen der vorhergehenden Ansprüche als Arzneimittel.

14. Verwendung einer Zusammensetzung nach einem der Ansprüche 1 bis 12 zur Herstellung eines Arzneimittels, das zur Behandlung und/oder Verhütung dermatologischer Erkrankungen bestimmt ist, die mit einer Störung der Verhornung einhergehen welche die Zelldifferenzierung und -vermehrung betrifft, insbesondere zur Behandlung von gewöhnlichen, mitesserbildenden, papeln- und pustelnbildenden, papeln- und mitesserbildenden, knoten- und zystenbildenden Akneformen, Conglobata-Akneformen, keloidartigen Akneformen im Nackenbereich, rezidivierenden Miliaris-Akneformen, nekrotischen Akneformen, Neonatorum-Akneformen, berufsbedingten Akneformen, Rosacea-Akneformen, senilen Akneformen, sonnenbedingten Akneformen und arzneimittelbedingten Akneformen.

15. Verwendung einer Zusammensetzung nach einem der Ansprüche 1 bis 12 zur Herstellung einer pharmazeutischen Zusammensetzung, die dazu bestimmt ist, gewöhnliche Akneformen zu verhüten und/oder zu behandeln.

16. Kosmetische Verwendung einer Zusammensetzung nach einem der Ansprüche 1 bis 12 zur Behandlung von Hauttypen, die zur Aknebildung neigen, oder zur Bekämpfung des fettigen Erscheinungsbilds der Haut oder des Haars.

17. Verfahren zur Formulierung einer Zusammensetzung, die einen Komplex umfasst, welcher sich aus einem Naphthoesäurederivat und aus Cyclodextrin und vorzugsweise aus Adapalen und aus Cyclodextrin, zusammensetzt, **dadurch gekennzeichnet, dass** es die folgenden Schritte umfasst:
Phase 1: Komplexbildungsschritt
1) Herstellen der Pulvermischung aus
a. dem Naphthoesäurederivat, vorzugsweise Adapalen:RAMEB-Cyclodextrin (1:n), wobei n die Molzahl von Cyclodextrin ist,
b. Zugeben von 10 % Wasser
2) Rühren
3) Bei 60 °C und 150 bar 2 Stunden lang reifen lassen
4) Trocknungsphase: über Nacht 50 °C unter Vakuum
Phase 2: Bildung der Komplexlösung
1) Abwiegen und Eintragen von gereinigtem Wasser in den Behälter
2) Abwiegen und Eintragen des freien RAMEB-Cyclodextrins, woraufhin weitergerührt wird, bis dieses in Lösung gegangen ist
3) Abwiegen des Adapalen/Cyclodextrin-Komplexes
4) Eintragen des Komplexes in die Lösung des freien RAMEB-Cyclodextrins
5) Rühren, um die Mischung zu homogenisieren
6) Nach Homogenisierung Filtrieren der Mischung über ein Filter mit einem Durchmesser zwischen 0,2 und 1 µm. Die Filtration ist nur dann vorschriftsmäßig, wenn die erhaltene Mischung klar ist
7) Die auf diese Weise erhaltene Komplexlösung muss 0,2 % (w/w) oder 0,6 % (w/w) Adapalen enthalten
Phase 3: Bildung der Formulierungsmatrix
Schritt a: Herstellung der wässrigen Phase
In ein Becherglas werden unter Rühren und erforderlichenfalls unter Erwärmen gereinigtes Wasser sowie der oder die Konservierungsstoffe und gegebenenfalls der oder die Gelbildner, der oder die Chelatbildner, der oder die hydrophilen Emulgatoren, der oder die Stabilisatoren, das oder die Feuchthaltemittel und/oder Emollientien eingetragen Mittel gegeben.
Die Mischung wird auf ungefähr 80 °C erwärmt.
Schritt b
In das obige Formulierungsbecherglas wird das freie RAMEB-Cyclodextrin im Überschuss gegeben, woraufhin weitergerührt wird, bis dieses in Lösung gegangen ist.
Schritt c (gegebenenfalls für eine Emulsion):
Hinzufügen der erforderlichen Menge der Komplexlösung, wie sie in der Phase 2 der vorliegenden Verfahrensweise erhalten wurde, zur obigen wässrigen Phase (erhalten in Schritt b).
Schritt d (gegebenenfalls für eine Emulsion): Herstellung der Fettphase:
Vermischen der lipophilen Emulgatoren, der ölartigen Verbindungen, der festen Fettsubstanzen sowie gegebenenfalls der lipophilen Emulgatoren, der Konservierungsstoffe. Die Mischung wird auf ungefähr 75 °C erwärmt.
Die Mischung wird erwärmt und homogenisiert, woraufhin als Letztes das flüchtige Silikon eingetragen wird, falls dieses in der Zusammensetzung vorliegt.
Schritt e (fakultativ): Emulgierung:
Bei einer Temperatur von 75 °C wird die Fettphase (Schritt d) unter Rühren in die wässrige Phase (Schritt c) eingetragen, um die Emulgierung durchzuführen.
Schritt f: Zusatz des Gelbildners (zur Herstellung eines Gels)
Der oder die Gelbildner werden unter Rühren in die Phase eingetragen, welche in b) erhalten wurde, um das Gel zu erhalten.
Es wird solange weitergerührt, bis vollkommene Homogenität erreicht ist.
Schritt g: Neutralisation
Erforderlichenfalls wird das Mittel zur Neutralisierung des Gelbildners eingetragen. Im Falle eines Gels wird dieses in die Phase eingetragen, welche in Schritt f) erhalten wurde. Im Falle einer Emulsion wird dieses der Phase zugesetzt, welche in Schritt e) erhalten wurde.
Schritt h: (fakultativ) Einstellung des Wassergehalts Erforderlichenfalls erfolgt eine Einstellung des Wassergehalts.
Schritt i (zur Herstellung eines Gels mit 0,1 % oder 0,3 % Adapalen): Zugabe der Komplexlösung
Phase 4: Fertigstellung
Nach dem Dispergieren sowie erforderlichenfalls dem Neutralisieren des Gelbildners werden gleiche Mengen der Zubereitung, welche in Schritt h) erhalten wurde, und der Komplexlösung, welche in Phase 2 hergestellt wurde, zugesetzt. Es wird solange weiter gerührt, bis der Ansatz homogen ist.

## Claims

1. Topical composition comprising, in a physiologically acceptable medium, at least one complex composed of a naphthoic acid derivative of formula (I), salts and esters thereof: 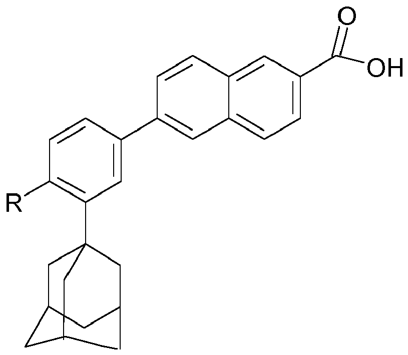 where R represents a hydrogen atom, a hydroxyl radical, a branched or unbranched alkyl radical having from 1 to 4 carbon atoms, an alkoxy radical having from 1 to 10 carbon atoms or a substituted or unsubstituted cycloaliphatic radical, the concentration of which derivative is between 0.001% and 10%, preferably between 0.01% and 5% and, more preferably, between 0.05% and 2% by weight of the total weight of the composition,
and of at least one methyl-β-cyclodextrin, said soluble molecular complex is obtained by the technology of dense fluids under pressure.

2. Composition according to Claim 1, **characterized in that** the dense fluid under pressure is supercritical CO₂.

3. Composition according to Claim 1, **characterized in that** the alkyl radical in formula (I) is the methyl, ethyl, propyl or butyl radical.

4. Composition according to Claim 1 or 3, **characterized in that** the alkoxy radical in formula (I) is the methoxy, ethoxy, propoxy, butoxy, hexyloxy or decyloxy radical.

5. Composition according to one of Claims 1 to 4, **characterized in that** the cycloaliphatic radical in formula (I) is the 1-methylcyclohexyl radical or the 1-adamantyl radical.

6. Composition according to one of Claims 1 to 5, **characterized in that** the compound derived from naphthoic acid is chosen from adapalene, 6-[3-(1-adamantyl)-4-hydroxyphenyl]-2-naphtoic acid, 6-[3-(1-adamantyl)-4-decyloxyphenyl]-2-naphtoic acid and 6-[3-(1-adamantyl)-4-hexyloxyphenyl]-2-naphtoic acid.

7. Composition according to Claim 6, **characterized in that** the compound derived from naphthoic acid is adapalene.

8. Composition according to Claim 1, **characterized in that** the methyl-β-cyclodextrin is chosen from TRIMEBs (heptakis(2,3,6-trimethyl)-β-CD), DIMEBs (heptakis(2,6-dimethyl)-β-CD) and also RAMEBs (Randomly Methylated β-cyclodextrin).

9. Composition according to Claim 8,**characterized in that** the methyl-β-cyclodextrin is that of RAMEB (Randomly Methylated β-cyclodextrin) type.

10. Composition according to one of Claims 1 to 9, **characterized in that** it is in the form of a gel.

11. Composition according to Claim 10, **characterized in that** the concentration of compound derived from naphthoic acid is equal to 0.1%.

12. Composition according to Claim 11, **characterized in that** the concentration of compound derived from naphthoic acid is equal to 0.3%.

13. Composition according to any one of the preceding claims, as a medicament.

14. Use of a composition according to one of Claims 1 to 12 for the preparation of a medicament for treating and/or preventing dermatological complaints associated with a keratinization disorder relating to cell differentiation and proliferation, especially for treating acne vulgaris, comedonal acne, papulopustular acne, papulocomedonal acne, nodulocystic acne, acne conglobata, acne keloid of the nape of the neck, recurrent miliary acne, acne necrotica, acne neonatorum, occupational acne, acne rosacea, senile acne, solar acne and acne medicamentosa.

15. Use of a compound according to one of Claims 1 to 12, for the preparation of a pharmaceutical composition for preventing and/or treating acne vulgaris.

16. Cosmetic use of a composition according to any one of Claims 1 to 12, for treating acne-prone skin or for combatting the greasy appearance of the skin or the hair.

17. Process for formulating a composition comprising a complex composed of a naphthoic acid derivative and cyclodextrin and preferably of adapalene and cyclodextrin, **characterized in that** it comprises the following steps:
Phase 1: Complexing step
1) Prepare the mixture of powder of
a. naphthoic acid derivative, preferably adapalene/RAMEB cyclodextrin (1/n), n being the number of moles of cyclodextrin
b. Add 10% water
2) Stir
3) Leave to mature for 2 hours at 60°C and 150 bar
4) Drying phase: 50°C under vacuum overnight
Phase 2: Formation of the complex solution
1) Weigh and introduce the purified water into the flask
2) Weigh and introduce the free RAMEB cyclodextrin, maintain stirring until dissolved
3) Weigh the adapalene/cyclodextrin complex
4) Introduce the complex into the solution of free RAMEB cyclodextrin
5) Stir in order to homogenize the mixture
6) Once homogenized, filter the mixture with a filter having a diameter between 0.2 and 1 µm. The filtration is correct only when the mixture obtained is clear
7) The complex solution thus obtained should contain 0.2% (w/w) or 0.6% (w/w) of adapalene
Phase 3: Formation of the formulary matrix
Step a: Preparation of the aqueous phase Introduced into a beaker, with stirring, if necessary at high temperature, are purified water and the preservative(s), and optionally the gelling agent(s), the chelating agent(s), the hydrophilic emulsifier(s), the stabilizer(s), the humectant(s) and/or emollient(s).
The mixture is brought to approximately 80°C.
Step b:
Introduce into the preceding formulary beaker the excess free RAMEB cyclodextrin, and maintain stirring until dissolved.
Step c (optionally for an emulsion):
Add the necessary amount of complex solution obtained during phase 2 of the present procedure to the preceding aqueous phase (obtained in step b).
Step d (optionally for obtaining an emulsion): Preparation of the fatty phase:
Mixing of the lipophilic emulsifiers, oily compounds, solid fatty substances and optionally of the lipophilic emulsifiers and preservatives. The mixture is brought to approximately 75°C.
The mixture is heated and after homogenization the volatile silicone is introduced last, if present in the composition.
Step e (optional): Emulsification:
At a temperature of 75°C, the fatty phase (step d) is introduced into the aqeuous phase (step c) while stirring in order to carry out the emulsification.
Step f: Addition of gelling agent (for the preparation of a gel)
The gelling agent(s) are introduced, with stirring, to the phase obtained in b) for the gel. The stirring is maintained until complete homogeneity is achieved.
Step g: Neutralization:
The neutralizing agent for the gelling agent is introduced, if necessary. In the case of a gel it will be introduced into the phase obtained in step f). For an emulsion, it will be introduced into the phase obtained in step e).
Step h: (optional) adjustment with water If necessary an adjustment with water is carried out.
Step i (for the preparation of a gel containing 0.1% or 0.3% of adapalene): Addition of the complex solution
Phase 4: completion
After dispersion and neutralization, if necessary, of the gelling agent, add, in equal amounts, the preparation obtained in step h) and the complex solution produced during phase 2. Stir until homogenized.
